# EUROPEAN PATENT SPECIFICATION

(11) **EP 1 629 729 B1**
(45) Date of publication and mention of the grant of the patent: **25.06.2008**
(21) Application number: 05016297.3
(22) Date of filing: 27.07.2005
(51) Int. Cl.: A23L 1/30, A23L 1/0522, A61K 31/738, A61P 3/08

(54) **Use of a crosslinked starch product**
Verwendung eines vernetzten Stärkeprodukts
Utilisation d'un produit à base d'amidon réticulé

(30) Priority: 29.07.2004 US 591997 P; 06.07.2005 US 175641
(43) Date of publication of application: 01.03.2006
(73) Proprietor: National Starch and Chemical Investment Holding Corporation, New Castle, Delaware 19720 (US)
(72) Inventor: Brown, Ian, Basking Ridge New Jersey 07920 (US); Okoniewska, Monica K., Princeton New Jersey 08540 (US); Billmers, Robert L., Stockton New Jersey 08559 (US); Skorge, Robert A., Somerville New Jersey 08876 (US)
(74) Representative: Held, Stephan

(56) References cited:
- EP-A- 0 506 166
- EP-A- 0 550 060
- EP-A- 1 088 832
- EP-A- 1 602 376
- US-A1- 2003 045 504
- US-A1- 2003 180 430
- US-B1- 6 749 880
- WOO K S ET AL: "CROSS-LINKED RESISTANT STARCH: PREPARATION AND PROPERTIES" CEREAL CHEMISTRY, AACC INTERNATIONAL, ST PAUL, MN, US, vol. 79, no. 6, November 2002 (2002-11), pages 819-825, XP001132662 ISSN: 0009-0352

## Description

### BACKGROUND OF THE INVENTION

The present invention relates to the use of a cross-linked or inhibited starch for preparation of an edible product for the control or regulation of blood glucose level. Such starches control and/or regulate the blood glucose level of mammals when used as a food or feed source by modifying the time and rate of post-prandial absorption.

Starch is a major source of energy in the typical western diet. Refined starches (for a description of refined starches see Imberty et al. Die Starke, 43 (10), 375-84 (1991)) are mostly eaten in the cooked form, which generally provides a high and rapid rise in blood glucose, being quickly and completely digested. However, some refined starches can resist enzymatic hydrolysis in the small intestine, such that the starch is not substantially broken down until it reaches the large intestine where it is utilized by resident microorganisms (this is defined as resistant starch or RS). Englyst (Englyst, H.N; et al. Eur J. Clin Nutr 46 (suppl.2):S33-S50 1992) defined three different categories of resistant starch related to their origin and means of resistance. A fourth type of RS was later described by Brown (Brown et al. Food Australia, 43(6), 272-75 (1995)) relating to chemically modified starches containing ethers, esters and cross-bonded starches that are resistant to enzymatic digestion.

The term available carbohydrate is defined as the total amount of carbohydrate in a food minus the amount of carbohydrate that is non-digestible. Non-digestible carbohydrates include dietary fiber, sugar alcohols and non-digestible sugars. The broad class of dietary fiber includes the group of starches defined above by Englyst and Brown (RS1 to 4). In some published examples, resistant starch is measured or quantified as dietary fiber (e.g. Chui et al. US Pat. # 5,902,410) using standard test methods (see AOAC 985.29 and 991.43) and provide little to no absorbable postprandial glucose, but are fermented in the large intestine. Furthermore, the presence of resistant starch affects the amount of available carbohydrates in the food serving in the same way dietary fiber (e.g., cellulose, inulin, psylium, and brans) affects the quantity of available carbohydrates.

Glycemic response (GR) refers to the differential effects of foods on blood glucose levels over the time period of 0 to 120 minutes (NIH Publication Number 99-3892 1999). It is measured as the incremental area under the blood glucose response curve in an individual subject for a particular food sample on a specific day. The magnitude and duration of the glycemic response to various foods reflects the variability in the rate and extent of the digestion and absorption of glucose containing components such as starch. This has been used to determine the magnitude of the postprandial glucose response to an individual food and also to compare (relative glycemic response) foods using the same sample or serving size. This is useful in determining the effects on blood glucose of foods as consumed by humans and animals.

As used in this application, glycemic index (Gl)(Jenkins, D.J.A. et al. Am J. Clin. Nutr. 34(3): 362-66 (1981)) is defined as "the incremental area under the blood glucose response curve of a 50 g available carbohydrate portion of a test food expressed as a percent of the response to the same amount of available carbohydrate in a standard food taken by the same subject". An arbitrary assignment of 100 is given for the standard food, which can either be 50 g of glucose or 50 g of white bread.

The Gl seeks to quantify the interactions of various ingredients in food and the role they play in how a carbohydrate source is digested and the glucose absorbed. By requiring a specified amount of available carbohydrate (50g) in the test food, a larger (sometime much larger) portion of the test food must be consumed. Alternatively stated, foods rich in fats, protein or dietary fiber would necessitate a larger serving size in order to ingest the required 50g of available carbohydrate.

As the food is ingested, the amount of glucose in the blood is subject to two basic mechanisms. The first is the rate of absorption into the blood stream of glucose as the food is digested. The second mechanism is the rate of absorption of the glucose from the bloodstream into the body tissue. Although this is a simplified view of these two mechanisms, one skilled in the art would recognize the complex and multifaceted nature of the mechanisms, reactions and processes involved. In normal healthy individuals, the body has mechanisms for regulating the blood glucose levels within certain specific ranges (fasting plasma glucose levels of 3.9 to 6.1 mmol/L as specified by the American Diabetes Association, Diabetes Care, 24(suppl), 1-9 (2001)). For example, increases in blood glucose levels stimulate the production of insulin, which amongst other functions facilitates the absorption of glucose into the tissue, but also exerts major functions in the metabolism of fats and proteins. Therefore, foods that cause an acute elevation in blood glucose concentration, have been shown to produce rapid (but offset) rises in serum insulin levels, which leads to the uptake, storage and use of glucose by the muscle cells, adipose tissue and the liver, consequently balancing the blood glucose concentration in the "normal" range.

Glucose that is absorbed into the tissue can be converted to glycogen as a means of storage for the muscles. Glycogen is used in times of physical activity and replenished in times of rest. Carb (carbohydrate) loading is a process athletes use to increase the store of energy in the form of glycogen in the muscle before an athletic activity. It is "a strategy in which changes to training and nutrition can maximize muscle glycogen stores prior to an endurance competition" (Michelle Minehan, AIS Sports Nutrition Program, 2003). Glycogen can also be transported from the muscle to the bloodstream to increase the bloods glucose levels if they.fall below certain levels.

A number of conditions are associated with over/under production of insulin or the reaction of cells in the body to the actions normally initiated by insulin. Insulin resistance (IR) is the condition in which the body tissue becomes less receptive to insulin and requires higher levels to achieve the same physiological effect. The principal effects of IR have been identified as decreased utilization of glucose by the body cells, resulting in increased mobilization of fats for the fat storage areas, and depletion of protein in the tissues of the body (Guyton, A.C., "Textbook of Medical Physiology (7th Ed.), W.B. Saunders Company: Philadelphia, Pa. 923-36). Other conditions arising from the over/under production of insulin include hypoglycemia, hyperglycemia, impaired glucose regulation, insulin resistance syndrome, hyperinsulinemia, dyslipidemia, dysfibrinolysis, metabolic syndrome, syndrome X and diabetes mellitus (type II also known as non-insulin depended diabetes mellitus (NIDDM)) and the physiological conditions that may arise such as cardiovascular disease, retinopathy, nephropathy, peripheral neuropathy and sexual dysfunction.

Another effect often associated with acute elevation and rapid swings in blood glucose levels is the inability to control and maintain body weight. Insulin, which plays many roles in the body, is also active in the conversion of glucose to fats (Anfinsen et al. US Patent Publication# 2004/0043106). Insulin resistance, necessitating higher levels of serum insulin, is thought to be a cause of weight gain as the increased insulin levels facilitate unnecessary fat storage. Experts have long recommended eating many small meals over the course of a day to attempt to regulate blood glucose (and the corresponding energy supply) at a constant, uniform level. Additionally, rapidly falling blood glucose levels (which normally happens after an acute elevation) have been shown to trigger a stimulation of appetite (hunger) in healthy adult humans. Alternatively, research indicates that glucose release over an extended time period leads to specific benefits which may include increased satiety for longer time periods (weight management such as weight loss and long term weight stabilization), sustained energy release (enhanced athletic performance including training), and improvements in mental concentration and memory.

A starch, or starch rich material, which could provide glucose to the blood over an extended time would serve to maintain normal/healthy blood glucose levels (i.e., normoglycemia) and reducing/eliminating rapid changes in blood glucose level. It would be an excellent carbohydrate source in the prevention and treatment of any of the conditions discussed above. Healthy individuals wishing to control glucose release or regulate the energy release from foods as well as the prevention or treatment of many diseases associated with irregularities in blood glucose and insulin concentrations could utilize foods containing these starches.

The preparation of cross linked resistant starch and the impact of this starch on glycemic index is disclosed in Woo at al., Cereal Chemistry Vol 79 , No.6 ,November 2002, pages 819-825

Surprisingly, it has now been discovered that crosslinked or inhibited starches may control and/or regulate the blood glucose level of mammals and post-prandial absorption. It has further been discovered that such crosslinked or inhibited starches, when properly formulated into foods or used as a supplement, may be used to provide the consumer with a controlled and/or regulated supply of glucose to the blood over an extended time period.

### SUMMARY OF THE INVENTION

The present invention relates to the use of a crosslinked or inhibited starch for preparation of an edible product to control and/or regulate the rate of glucose release from foods or supplements after consumption (i.e., post-prandial). Such starches include those prepared by treating native starch or starch rich materials such as flour or grits using methods known in the art to crosslink or inhibit starch, specifically those treated with sodium trimetaphosphate, and/or sodium tripolyphosphate. Such crosslinked or inhibited starches, may be capable of reducing the initial acute elevation of blood glucose, and when properly formulated into foods, may be used to provide the consumer with controlled/regulated glucose over an extended time period and assist in providing normal/healthy blood glucose levels, including those individuals who may develop insulin resistance.

Granular, as used herein, is intended to mean not gelatinized or dispersed by any chemical or physical process. Granular starches can be determined using microscopy by the presence of birefringence (Maltese cross) under polarized light. Granular starches are also not significantly soluble in water below their gelatinization temperature. Non-granular starches are those that have been treated or processed to be readily soluble in water (CWS) below their gelatinization temperature (typically about 65°C). Some starches can be processed to become soluble and then are allowed to retrograde so as to form particles (crystallites) that are no longer soluble below 100°C, but are also not granular. In one embodiment of this invention, the granular form of starch was used.

Most researchers and publications have chosen two points in time to measure the digestibility of carbohydrates. These points are at 20 and 120 minutes after ingestion or for *in vitro* techniques after the enzyme digestion commences, but do not accurately reflect the absorption in the stomach and small intestines. For purposes of this application, digestion of various samples have been measured at 20, 120 and 240 minutes to better relate to the true physiological effects these samples will have in the mammalian digestive system.

As used herein, the term rapidly digestible starch is intended to mean a starch or portions thereof which are fully absorbed within the first 20 minutes after ingestion as measured by the rapid increase in blood glucose concentration (Englyst et al., Eur. J. Nutr. 46(suppl. 2), s33-s50).

As used herein, the term resistant starch is intended to mean a starch, or the fraction thereof, which is not digested in the small intestines.

The term slowly digestible starch is intended to mean a starch, or the fraction thereof, which is neither rapidly digestible starch nor resistant starch. Alternatively stated, slowly digestible starch is any starch that releases a substantial portion of its glucose to the mammalian body over the entire length of the stomach and small intestines (typically between 20 minutes and 240 minutes in humans). For a more complete description of these starches see Englyst et al, European Journal of Clinical Nutrition, 1992, 46,S33-S50. (Note: Englyst describes slowly digestible starches as those that release their glucose between 20 and 120 minutes as opposed to between 20 and 240 minutes.)

As used herein, the term bound phosphorous is intended to mean the bound phosphorus, as determined by the test set forth in the Examples section and added bound phosphorus is intended to mean the bound phosphorus which is not naturally present in the starch and is added on by chemical or other means. Added bound phosphorus would therefore be determined by subtracting the bound phosphorus of the unmodified starch base from that of the modified starch.

### BRIEF DESCRIPTION OF THE DRAWINGS

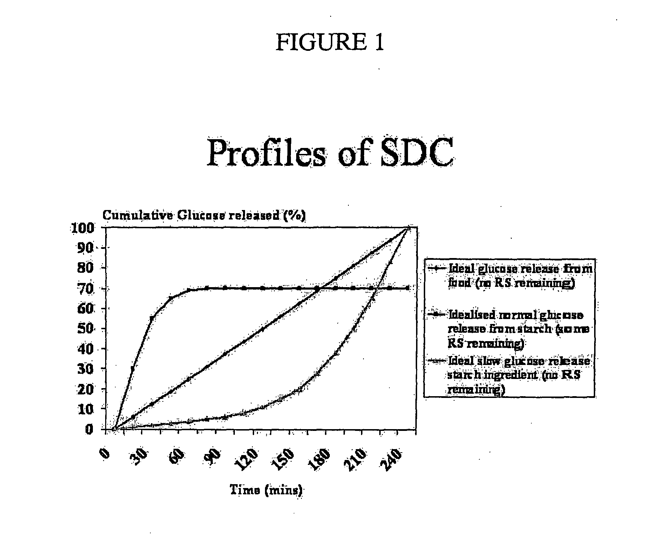

Figure 1 depicts the ideal slow glucose release compared to that of normal starches and the ideal glucose release from foods containing such starches.
Figure 2 depicts the actual glucose release of uncooked corn starches crosslinked to various levels with STPP/STMP.

### DETAILED DESCRIPTION OF THE INVENTION

The present invention relates to the use of a crosslinked or inhibited starch for preparation of an edible product to control and/or regulate the blood glucose level of mammals and post-prandial absorption. Such starches are prepared by treating native starch or starch rich materials such as flour or grits using methods known in the art to crosslink or inhibit starch, specifically with sodium trimetaphosphate (STMP) and/or sodium tripolyphosphate (STPP). Such crosslinked or inhibited starches, when properly formulated into foods or taken as a supplement, may be used to provide the consumer with more constant blood glucose (prevent/minimize acute elevation) levels over an extended time period (corresponding to the time the material is in the stomach/small intestine) than would be possible with other types of starches. Such starches and foods containing these starches will help the consumer regulate and maintain normal and healthy blood glucose levels.

Starch, as used herein, is intended to include all starches, flours and starch containing materials derived from tubers, grain, legumes and seeds or any other native source, any of which may be suitable for use herein. A native starch as used herein, is one as it is found in nature. Also suitable are starches derived from a plant obtained by standard breeding techniques including crossbreeding, translocation, inversion, transformation or any other method of gene or chromosome engineering to include variations thereof which are typically referred to as genetically modified organisms (GMO). In addition, starch derived from a plant grown from artificial mutations (including those from chemical mutagens) and variations of the above generic composition, which may be produced by known standard methods of mutation breeding, are also suitable herein.

Typical sources for the starches are cereals, tubers, roots, legumes and fruits. The native source can be corn (maize), pea, potato, sweet potato, banana, barley, wheat, rice, oat, sago, amaranth, tapioca (cassava), arrowroot, canna, triticale, and sorghum, as well as waxy (low amylose) varieties thereof. Particularly useful sources include maize, potato, cassava, and rice. As used herein, the term "waxy" or "low amylose" is intended to include a starch containing no more than about 10%, particularly no more than about 5%, most particularly no more than about 2%, by weight amylose. The invention embodied within relates to all starches and is intended to include all starch sources, including those which occur in nature, are genetically altered, or are obtained from hybrid breeding. However, high amylose starches, that is starches with greater than 40% amylose, are not suitable for use in this invention.

The starch of this invention is prepared by treating native starch or starch rich materials with multifunctional (i.e., difunctional) crosslinking reagents. In one embodiment, the reagent is selected from the group consisting of sodium trimetaphosphate, sodium tripolyphosphate, and combinations thereof. Such crosslinking modifications are known in the art and are described for example in Modified Starches: Properties and Uses, Ed. Wurzburg, CRC Press, Inc., Florida (1986). One skilled in the art would recognize that by varying the reaction conditions and reagents, it may be possible to vary the level and ratio of disubstitution vs. mono-substitution. How this ratio affects the rate of digestions and absorption into the body is dependent upon many factors including starch type, amylose content, and granular composition/conformation as well as reagent type, and reaction conditions. The rate of digestion is also dependent on the way or manner in which the food is prepared and the reaction of the individual to such foods, including variations in each individual's biochemistry and physiology.

The amount of crosslinking is dependent, *inter alia,* upon the starch, the crosslinking reagent and whether the starch will be cooked out prior to ingestion. When crosslinking reagent such as STMP is used, the amount of crosslinking is measured by the bound phosphorous content and, in this invention, is present in a slowly digestible effective amount. The amount of bound phosphorous is in the range of 0.10 to 0.35% bound phosphorous.

The starch is further modified by oxidation or dextrinization to provide desirable textural and/or physical properties. The modification may be accomplished before or after the crosslinking/inhibition depending upon the type of additional modification. It would be within the knowledge of the skilled artisan as to what combinations are possible and in what order such modification may be accomplished. Additional modifications may include molecular weight reduction such as acid conversion and/or enzyme treatment, and substitution with propylene oxide (PO), ethylene oxide (EO), octenylsuccinic anhydride (OSA), acetylation.

Modifications as described above are typically accomplished in aqueous media with some form of pH control or pH adjustment. A skilled practitioner would readily appreciate the variety of materials and equipment for carrying out these reactions. For a review of these reaction conditions, see Modified Starches: Properties and Uses, Ed. Wurzburg, CRC Press, Inc., Florida (1986), chapter 4. Other reaction media and conditions may be utilized and will provide materials under the scope of the invention. These include, but are not limited to, dry heat reactions, solvent reactions, supercritical fluid reactions and gaseous conditions.

The starches may be modified in the granular state or after gelatinization using techniques known in the art. Such techniques include those disclosed for example in U.S. Patent Nos. 4,465,702, 5,037,929, 5,131,953, and 5,149,799. Also see, Chapter XXII- "Production and Use of Pregelatinized Starch", Starch: Chemistry and Technology, Vol. III- Industrial Aspects, R.L. Whistler and E.F. Paschall, Editors, Academic Press, New York 1967.

The starches of this invention may be converted, such as fluidity or thin-boiling starches prepared by oxidation, acid hydrolysis, enzyme hydrolysis, heat and/or acid dextrinization. These processes are well known in the art.

The starch may be purified by any method known in the art to remove starch off-flavors, colors, or sanitize microbial contamination to insure food safety or other undesirable components that are native to the starch or created during processing. Suitable purification processes for treating starches are disclosed in the family of patents represented by EP 554 818 (Kasica, et al.). Alkali washing techniques are also useful and described in the family of patents represented by U.S. 4,477,480 (Seidel) and 5,187,272 (Bertalan et al.). The starch may be purified by enzymatic removal of proteins. Reaction impurities and by-products may be removed by dialysis, filtration, centrifugation or any other method known in the art for isolating and concentrating starch compositions. The starch may be washed using techniques known in the art to remove soluble low molecular weight fractions, such as mono- and di-saccharides and/or oligosaccharides.

The resultant starch is typically adjusted to the desired pH according to its intended end use. In general, the pH is adjusted to 3.0 to about 6.0. In one embodiment, the pH is adjusted to 3.5 to about 4.5, using techniques known in the art.

The starch may be recovered using methods known in the art, including without limitation by filtration or by drying, including spray drying, freeze drying, flash drying or air drying.

The resultant starch has an altered digestion profile, such that less than 25% is digested within the first 20 minutes of ingestion. In one embodiment, the starch is less than 20% digested, and in another embodiment less than 10% digested, within the first 20 minutes.

Further, the resultant starch is 30 to 70% digested within 120 minutes of ingestion. In one embodiment, the starch is at least 40-60% digested within 120 minutes of ingestion and in another embodiment, at least 45-55% is digested within 120 minutes.

In addition, the resultant starch is at least 60% digested within 240 minutes of ingestion. In one embodiment, the starch is at least 70% digested within 240 minutes of ingestion and in another embodiment, is at least 80% digested within 240 minutes and in yet another embodiment, is at least 90% digested within 240 minutes.

It would be apparent to one skilled in the art that cooking a starch will affect the digestibility and rate of absorption of the glucose into the blood stream. For a review of the effect of cooking see Brown, M.A., et al. British Journal of Nutrition, 90, 823-27 (2003).

In a recent patent application, Brown et al., US 2003/0045504A1 published March 6, 2003, the relationship between resistant starch and other components in the foods, such as various lipids, have an effect on the digestibility, glycemic index (GI), glycemic response (GR) and blood glucose levels.

Starch is rarely consumed on its own, but is typically consumed as an ingredient in a food product. This food product may be manipulated to result in desired glucose release curves. In one embodiment, the food is manipulated to provide a substantially zero order glucose release curve, to provide an essentially constant and sustained glucose release rate.

Starch and starch rich materials such as flours may be consumed in their raw state, but are typically consumed after cooking and/or other processing. Therefore, the invention is intended to include those starches which, when added to food and processed, have the advantage of changing the glucose release curve. In one embodiment, the food containing the processed starch provides a substantially zero order glucose release curve, to provide an essentially constant and sustained glucose release rate. Such foods are modeled by the methods described in the Examples section, *infra.*

The crosslinked starch of this invention does not produce a large rapid increase in blood glucose levels typical of a high glycemic index starch, such as most native starches, but instead provides a more moderate increase above the baseline which is sustained for a longer time period. It may also be process tolerant in that there is no large and rapid increase in blood glucose levels after ingestion of food containing the starch and the glucose release from the prepared and/or processed food is substantially constant.

The crosslinked or inhibited starch of this invention may be used in a variety of edible products including, but not limited to: baked goods, including crackers, breads, muffins, bagels, biscuits, cookies, pie crusts, and cakes; cereal, bars, pizza, pasta, dressings, including pourable dressings and spoonable dressings; pie fillings, including fruit and cream fillings; sauces, including white sauces and dairy-based sauces such as cheese sauces; gravies; lite syrups; puddings; custards; yogurts; sour creams; beverages, including dairy-based beverages; glazes; condiments, confectioneries and gums; and soups.

Edible products also is intended to include nutritional foods and beverages, including dietary supplements, diabetic products, products for sustained energy release such as sports drinks, nutritional bars and energy bars.

The crosslinked or inhibited starch may be also used in a variety of animal feed products, weaning formulations affording desirable growth and development of the post weaned animal, pharmaceutical formulations, nutraceuticals, over the counter (OTC) preparations, tablets, capsules and other known drug delivery vehicles for human and/or animal consumption and/or any other applications that can benefit from constant release of glucose from the formulation.

The crosslinked or inhibited starch may be added in any amount desired or necessary to obtain the functionality of the composition. In one embodiment, the starch may be added in an amount of from 0.01% to 99% by weight of the composition. In another embodiment, the starch is added in an amount of from 1 to 50%, by weight of the composition. The starch may be added to the food or beverage in the same manner as any other starch, typically by mixing directly into the product or adding it in the form of a sol. The edible product may contain additional components, for example water.

Edible products may be formulated using crosslinked or inhibited starch which will provide a substantially zero order glucose release rate. Such products may provide the consumer with glucose over an extended time period and more constant blood glucose levels.

Products which control and/or regulate the rate and magnitude of glucose adsorption may increase satiety for longer time periods, and thus be useful in weight management. They may also provide sustained energy release, and thus enhance athletic performance including training, and improvements in concentration maintenance and memory.

The products may also provide pharmaceutical benefits, including reducing the risk of developing diabetes, treating obesity such as weight loss or weight management, and preventing or treating hyperglycemia, insulin resistance, hyperinsulinemia, dyslipidemia, and dysfibrinolysis.

### EXAMPLES

The following comparative examples are presented.

### The following test procedures are used throughout the examples:

Simulated Digestion - (Englyst et al., European Journal of Clinical Nutrition, 1992, 46,S33-S50)

Food samples are ground/minced as if masticated. Powder starch samples are screened to a particle size of 250 microns or less. A 500-600 mg ± 0.1 mg of sample is weighed and added to the sample tube. 10 ml of a pepsin (0.5%), guar gum (0.5%), and HCl (0.05 M) solution is added to each tube.

Blank and glucose standard tubes are prepared. The blank is 20 ml of a buffer containing 0.25 M sodium acetate and 0.02% calcium chloride. Glucose standards are prepared by mixing 10 ml sodium acetate buffer (described above) and 10ml of 50 mg/ml glucose solution. Standards are prepared in duplicate.

The enzyme mix is prepared by adding 18 g of porcine pancreatin (Sigma P-7545) to 120 ml of deionized water, mixing well, then centrifuging at 3000g for 10 minutes. The supernatant is collected and 48mg of dry invertase (Sigma I-4504) and 0.5 ml AMG E (Novo Nordisk) are added.

The sample tubes are pre-incubated at 37°C for 30 min, then removed from the bath and 10 ml of sodium acetate buffer is added along with glass balls/marbles (to aid in physical breakdown of the sample during shaking).

5 ml of the enzyme mixture is added to the samples, blank, and standards. The tubes are shaken horizontally in a 37°C waterbath at approximately 180 strokes/min. Time "zero" represents the first addition of the enzyme mixture to the first tube.

After 20, 120, and 240 minutes, 0.5-ml aliquots are removed from the incubating samples and placed into a separate tube of 20ml 66% ethanol (to stop the reaction). After 1 hour, an aliquot is centrifuged at 3000g for 10 minutes.

The glucose concentration in each tube is measured using the glucose oxidase/peroxidase method (Megazyme *Glucose* Assay *Procedure* GLC9/96). This is a colorimetric procedure.

The degree of starch digestion is determined by calculating the glucose concentration against the glucose standards, using a conversion factor of 0.9. Results are given as "% starch digested" (dry weight basis) after 20, 120, and 240 minutes. The experimental error of the test was determined to be ±4.

Every sample analysis batch includes a reference sample of uncooked cornstarch. The accepted range of % digestion values for cornstarch are:

| Time (minutes) | 20 | 120 | 240 |
|---|---|---|---|
| Sample 1 (control)¹ | 18 ± 4 | 80 ± 4 | 90 ± 4 |

| | | | |
|---|---|---|---|
| ¹Melogel® starch, cornstarch commercially available from National Starch and Chemical Company, Bridgewater, NJ, USA. | | | |

### Bound Phosphorus Analysis

Prepare 1.7% slurry of starch in 5% EDTA solution and stir for 5min and filter. Wash the sample on the filter with 200 ml of deionized water four times. Dry sample at room temperature. Prepare quantitatively 3% starch slurry in 4N HCl, add boiling stones, and boil the sample for 7min, cool to room temperature, quantitatively dilute with deionized water, centrifuge to remove any possible particulate. The sample is then analyzed by Inductively Coupled Plasma Spectrometry (ICP) for phosphorus using standard analytical procedures to obtain total bound phosphorus. Added bound phosphorus is determined by subtracting total bound phosphorus of the unmodified starch from that of the modified starch.

### Model Cookie / Biscuit Food System

A procedure described below was followed to model preparation of cookies.
Measure moisture of experimental starch gravimetrically.
Calculate amount of additional water required to adjust the starch to a moisture content of 25% (w/w) which is a typical moisture level for cookie and biscuit dough.
Weigh 50g of starch into a mixing bowl of a Sunbeam Mixmaster, lower mixing blades into a bowl and turn the mixer on to a 'fold' position.
Begin addition of pre-calculated amount of water by spraying the water onto the starch while mixing to ensure even moisture distribution. Complete water addition in 5 min.; continue mixing on 'fold' setting until starch does not stick to walls of the mixing bowl. The total mixing time is 8-10min.
Transfer 50g of the hydrated starch into an aluminum tin (145mm x 120mm x 50mm) and spread evenly to cover the entire bottom of the pan.
Preheat an oven to 190°C.
Bake the hydrated starch at 190°C for 20 min.
Take the starch out from the oven, place immediately in 4oz (118.3 ml) plastic jar and close the lid.
Cool the starch to room temperature and determine moisture of baked starch gravimetrically. The moisture content of the baked starch should be in a 5-8% (w/w) range which is typical for cookies and biscuits.
Test glucose release from starch immediately or store it in an air-tight container for testing the following day.

### Example 1 - Preparation of Crosslinked Starches

*Sample 1 -* control corn starch; Melogel® starch, commercially available from National Starch and Chemical Company, Bridgewater, NJ, USA
*Sample 2 -* 3,000 ml of tap water were measured into a reaction vessel. 100g Na₂SO₄ were added with agitation and stirred until dissolved. With good agitation, 2,000g corn starch were added and then 3% NaOH was added drop-wise to the slurry as needed to reach 40ml alkalinity (actual 667g NaOH for 44.00ml alkalinity). The slurry was stirred 1 hr and the pH was recorded (pH 11.68). The temperature was adjusted to 42°C. 160g of a 99/1 STMP/STPP blend was added and allowed to react for 4 hours. The final pH and temperature were recorded (pH 11.02 and 42°C). The pH was adjusted to 5.5 with 3:1 HCI (pH 5.47 using 164.99g HCI). The resultant starch cake was filtered and washed twice with 3,000 ml tap water. The cake was crumbled and air dried.
*Sample 3 -* 3,000 ml of tap water was measured into a reaction vessel. 100g Na₂SO₄ were added with agitation and stirred until dissolved. With good agitation, 2,000g corn starch were added and then 3% NaOH was added drop-wise to the slurry as needed to reach 40ml alkalinity (667g NaOH for 44.00ml alkalinity). The slurry was stirred 1 hr and the pH was recorded (pH 11.69). The temperature was adjusted to 42°C. 160g of a 99/1 STMP/STPP blend was added and allowed to react for 17 hours. The final pH and temperature were recorded (pH 11.32 and 42°C). The pH was adjusted to 5.5 with 3:1 HCI (pH 5.57 using 146.88g HCI). The resultant starch cake was filtered and washed twice with 3,000 ml tap water. The cake was crumbled and air dried.
*Sample 4 -* 3,300 ml of tap water was measured into a reaction vessel. 110g Na₂SO₄ were added with agitation and stirred until dissolved. With good agitation, 2,200g corn starch were added and then 3% NaOH was added drop-wise to the slurry as needed to reach 40ml alkalinity (733g NaOH for 44.14ml alkalinity). The slurry was stirred 1 hr and the pH was recorded (pH 11.71). The temperature was adjusted to 42°C. 220g of a 99/1 STMP/STPP blend was added and allowed to react for 17 hours. The pH was maintained with a controller and 3% NaOH (556.6g consumed). The final pH and temperature were recorded (pH 11.19 and 42°C). The pH was adjusted to 5.5 with 3:1 HCl (pH 5.49 using 285.38g HCl). The resultant starch cake was filtered and washed twice with 3,300 ml tap water. The cake was crumbled and air dried.
*Sample 5 -* 2,500 pounds (1134kg) of tap water were measured into a reaction vessel. 100 lbs (45.4kg) Na₂SO₄ were added with agitation and stirred until dissolved. With good agitation, 2,000 lbs (907.2kg) of corn starch were added. Then 3% NaOH was added at 4lbs/minute (1.8kg/minute) to the starch slurry as needed to reach 40ml alkalinity (about 600 lbs (272.2kg) NaOH for 46 ml alkalinity). The mixture was stirred for 1 hr and the pH recorded (pH 11.6). Temperature was adjusted to 108°F (42°C). 200 lbs (90.7kg) of a 99/1 STMP/STPP blend were added and reacted for 17 hours. The final pH and temperature were recorded (pH 11.4 and 108 °F (42°C)). pH was adjusted to 5.5 with 3:1 HCl as needed (pH 5.4 using 75 lbs. HCl (34kg)). The starch was washed and centrifuged on a Merco centrifuge and flash dried.
*Samples 8, 9, 11, 13, 14, 15 and 16* were prepared by the same procedure as sample 3. The amount of 99/1 STMP/STPP blend was adjusted to results in a desired bound phosphorus level.
*Sample 18 -* 750ml of water was measured into reaction vessel. 2.5g of NaCl were added with agitation and stirred until dissolved. 500 g of starch were added to the salt solution. 3% NaOH was added drop-wise to the slurry with strong agitation as needed to reach pH 11-11.5. The slurry was stirred 1 hr and the pH was recorded (pH 11.43). 20g of POCl₃ was added and allowed to react for 30min while stirring at room temperature. The pH was adjusted to 5.5 with 3:1 HCl. The resultant starch cake was filtered and washed twice with 750 ml tap water. The cake was crumbled and air dried.

The amount of bound phosphorus and the amount of glucose released were determined for each of the uncooked starch samples. The results are listed in Table I, below.

**Table I**

| Starch Base | Sample ID | STMP/STPP | Bound Phosphorus | Glucose Released O/T (%) | | |
|---|---|---|---|---|---|---|
| | | (% on starch) | (%) | 20min | 120min | 240min |
| Dent corn | 1 | Native | 0.04 | 17 | 75 | 85 |
| Dent corn | 2 | 8 | 0.12 | 17 | 71 | 80 |
| Dent corn | 3 | 8 | 0.21 | 9 | 48 | 62 |
| Dent corn | 4 | 10 | 0.31 | 1 | 8 | 15 |
| Dent corn | 5 | 12 | 0.40 | 0 | 2 | 4 |

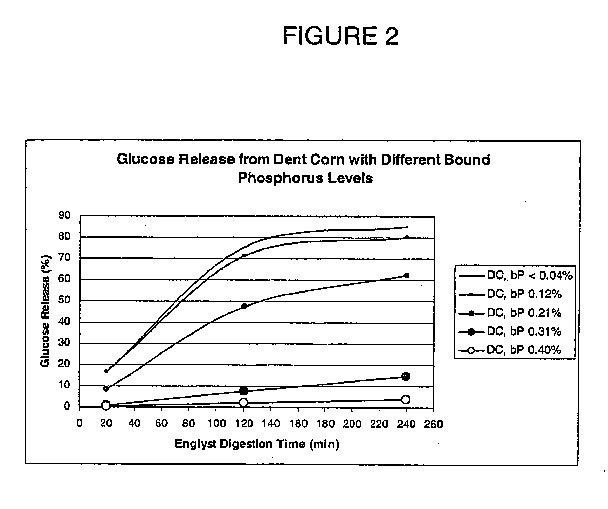

As can be seen from Table I, Sample 3 shows that starch may be crosslinked using a combination of STMP and STPP to result in the altered digestion curve of this invention. The digestion curves of these starches are depicted in Figure 2.

### Example 2 - Glucose Release in Model Food Systems

A variety of base starches were modified using STMP/STPP according to the general procedure of Example 1 to obtain a variety of total bound phosphorus levels. The digestibility of these starches were tested, either as is or in model food systems.

The results are listed in Table II, below.

| Sample # | Base Starch | Total Bound Phosphorus (%) | Model | T=20 min | T=120 min | T=240 min |
|---|---|---|---|---|---|---|
| | | | | | | |
| 1 | Dent | N/A | N/A | 18 | 80 | 85 |
| 2 | Dent | N/A | Cookie | 29 | 73 | 80 |
| 3 | Dent | 0.24 | N/A | 1 | 27 | 60 |
| 4 | Dent | 0.12 | Cookie | 19 | 65 | 75 |
| 5 | Dent | 0.14 | Cookie | 14 | 47 | 56 |

| Sample # | Base Starch | Total Bound Phosphorus (%) | Model | T=20 min | T=120 min | T=240 min |
|---|---|---|---|---|---|---|
| | | | | | | |
| 6 | High (~70%) Amylose | N/A | N/A | 11 | 26 | 30 |
| 7 | High (~70%) Amylose | N/A | Cookie | 9 | 23 | 27 |
| 8 | High (~70%) Amylose | 0.23 | N/A | 6 | 13 | 16 |
| 9 | High (~70%) Amylose | 0.25 | Cookie | 7 | 16 | 18 |
| | | | | | | |
| 10 | Tapioca | N/A | N/A | 9 | 42 | 52 |
| 11 | Tapioca | 0.15 | Cookie | 14 | 46 | 58 |
| | | | | | | |
| 12 | Waxy com | N/A | N/A | 35 | 94 | 100 |
| 13 | Waxy corn | 0.31 | Cookie | 17 | 50 | 60 |
| 14 | Waxy corn | 0.41 | Cookie | 12 | 31 | 36 |
| | | | | | | |
| 15 | Rice | 0.17 | N/A | 17 | 55 | 68 |
| | | | | | | |
| 16 | Wheat | 0.21 | N/A | 22 | 69 | 82 |

As can be seen from Table II, a variety of starch bases may be crosslinked using a combination of STMP and STPP to result in the altered digestion curve of this invention in model food systems.

### Example 3 - Comparison of Crosslinkinq Reagents

The digestion of corn modified with STMP/STPP from Example 1 was compared with that of corn modified with phosphorus oxychloride. The results are shown in Table III, below.

**Table III**

| Sample No. | Sample | Bound P (%) | T=20 (% GR) | T=120 (% GR) | T=240 (% GR) |
|---|---|---|---|---|---|
| | | | | | |

| GRANULAR STARCH | | | | | |
|---|---|---|---|---|---|
| 1 | Dent Corn | na | 18 | 80 | 90 |
| 17 | Dent Corn STMP/STPP | 0.26 | 1 | 27 | 58 |
| 18 | Dent Corn POCl₃ | 0.27 | 0 | 2 | 2 |
| | | | | | |

| MODEL COOKIE | | | | | |
|---|---|---|---|---|---|
| 19 | Dent Corn STMP/STPP | 0.26 | 5 | 39 | 63 |
| 20 | Dent Corn POCl₃ | 0.27 | 3 | 11 | 16 |

As can be seen from Table III, the dent corn product, either as is or in the model cookie, did not obtain the digestion curves of the present invention when modified with phosphorus oxychloride in the range of bound phosphorus claimed.

### Example 4 - Food Products Containing Starch

The starch samples of Example 1 are added to at levels of 5-40% to replace flour or other carbohydrate ingredients in six different food products. All ingredients are listed as weight % of the formulation.
1) White Pan Bread
2) Semolina Pasta
3) Nutrition Bar
4) Flavored Yogurt Drink
5) Tea Biscuit
6) Cereals

### 1) White Pan Bread

| | |
|---|---|
| Patent Flour | 55.6 |
| White Granulated Sugar | 4.3 |
| Shortening | 2.8 |
| lodized Salt | 1.1 |
| Active Dry Yeast | 0.6 |
| Dough Conditioner | 35.0 |
| Water | 0.6 |
| Total | 100.0 |

### Preparation:

Combine all ingredients and water in Hobart mixer. Mix on low speed for 2 minutes. Mix on Medium speed for 14 minutes. Allow dough to rest 5 minutes. Scale dough to loaves (510g for ½ kg Loaves). Allow dough to rest 5 minutes. Mold loaves in Glimek Dough-molder. Proof at 90% RH, 80°C. Bake at 210°C for 22 minutes.

### 2) Semolina Pasta

| | |
|---|---|
| Semolina Flour | 74.1 |
| Water | 23.3 |
| Dried Egg Whites | 1.5 |
| Dough Conditioner | 1.1 |
| Total | 100.0 |

### Preparation:

Combine all ingredients and water in Hobart/Kitchen Aid mixer. Mix on low speed for 10 minutes.
Feed into sheeter to form into noodles. Cook by placing noodles in boiling water for 5-10 minutes with stirring. Drain water

### 3) Nutrition Bar

| | |
|---|---|
| Protein Powder | 33.6 |
| Brown Rice Syrup | 21.3 |
| Dry Oats | 10.5 |
| Honey | 9.0 |
| Nonfat Dry Milk | 9.7 |
| Soy Oil | 2.8 |
| Peanut Flour | 5.3 |
| Apple Sauce or Raisin Paste | 7.8 |
| Total | 100.0 |

### Preparation:

Combine all dry ingredients (except oats) in Hobart mixer. Mix on low speed for 5 minutes, or until blended. Continue mixing while adding liquid ingredients. Fold in oats while continuing to mix at low speed. Form bar into desired shape by pressing into a form.

### 4) Flavored Yogurt Drink

| | |
|---|---|
| Whole Milk | up to 100.0 |
| Starter culture (Danisco's Jo-mix NM 1-20) | |
| Nonfat Dry Milk | optional |
| Total | 100.0 |

### Yogurt Preparation:

Preheat milk to 65°C. Homogenize at 10.34 megapascal, then hold for 2 minutes at 93°C. Cool mix to 44°C. Inoculate with starter culture. Incubate until pH reaches 4.5 then cool to 4.5°C. Yogurt may be pumped to smooth curd.

| Juice mix | |
|---|---|
| Water | 47.5 |
| Strawberry conc. (40-60 brix) | 40.0 |
| Fructose | 10.0 |
| Pectin | 2.5 |
| Total | 100.0 |

### Juice Preparation:

Dry blend fructose and pectin. Add dry mix, water, and strawberry concentrate to a blender. Blender until fructose and pectin are dispersed. Cook juice mix in a hot water bath at 80°C for 15 minutes. Cool to 4.5°C.

### Final Product Preparation:

Blend Yogurt and Juice Mix at a ration of 9:1. Co-Homogenize at megapascals of 17.3/3.5 (two stages). Store finished product at 4.5°C

### 5) Tea Biscuit

| | |
|---|---|
| Wheat Flour | 48.0 |
| White Granulated Sugar | 20.5 |
| Whey Powder | 1.3 |
| Baking Powder | 1.2 |
| Salt | 0.6 |
| Shortening | 9.6 |
| Egg Yolks | 2.0 |
| Water | 16.8 |
| Total | 100.0 |

### Preparation:

Combine all dry ingredients and shortening in a Hobart mixer. Mix on low for 5 minutes. Add egg yolks and water. Mix on low for 5 minutes. Roll or sheet dough and cut biscuits. Bake at 176°C for 12-15 minutes.

### 6) Cereal

### a) Extruded breakfast cereal (maize based)

| | |
|---|---|
| Modified maize starch or flour | 40.0% |
| Maize polenta | 45.0% |
| Sugar | 10.0% |
| Salt | 2.0% |
| Malt | 3.0% |
| | 100.0% |

### b) Extruded breakfast cereal (multigrain)

| | |
|---|---|
| Modified maize starch or flour | 43.0% |
| Rice flour | 11.5% |
| Oat flour | 11.5% |
| Wheat flour | 20.4% |
| Sugar | 9.0% |
| Malt | 2.6% |
| Salt | 2.0% |
| | 100.0% |

### Preparation:

The cereals are prepared using methods known in the art. They are extruded, flaked and toasted or extruded and expanded). The cereals are further dried, if necessary, to a final mositure content less than 3%.

The foods are digested using Englyst digestion method and glucose release is monitored over 20, 120 and 240min. The release of glucose is substantially linear over the digestion time.

## Claims

1. Use of a crosslinked starch with an added bound phosphate content of 0.10 to 0.35% for preparation of an edible product for controlling the blood glucose level of a mammal, wherein said starch provides less than 25% of the glucose release at 20 minutes, between 30-70% at 120 minutes and greater than 60% at 240 minutes, and wherein said starch is not a high amylose starch, that is starch with greater than 40% amylose, the starch is in the granular form and where by glucose release is measured according to the simulated digestion method as disclosed in the description, and the starch has been dextrinized or oxidized..

2. Use according to claim 1, wherein the starch provides less than 20% of the glucose release at 20 minutes.

3. Use according to claim 1, wherein the starch provides less than 10% of the glucose release at 20 minutes.

4. Use according to claim 1, wherein the starch provides between 40-60% of the glucose release at 120 minutes

5. Use according to claim 1, wherein the starch provides between 45-55% of the glucose release at 120 minutes.

6. Use according to claim 1, wherein the starch provides greater 70% of the glucose release at 240 minutes.

7. Use according to claim 1, wherein the starch provides greater 80% of the glucose release at 240 minutes.

8. Use according to claim 1, wherein the starch provides greater 90% of the glucose release at 240 minutes.

9. Use according to claim 1, where the starch is crosslinked using a reagent selected from sodium trimetaphosphate, sodium tripolyphosphate, and a combination thereof.

10. Use according to claim 1, wherein the starch is present in the edible product in an amount of 5-40% dry weight basis.

11. Use according to claim 1, wherein the glucose release from the food is substantially zero order.

12. Use according to claim 1, wherein the glucose release rate is substantially constant over the first 240 minutes.

13. Use according to claim 1 wherein a regulated supply of glucose to a mammal is provided.

## Patentansprüche

1. Verwendung einer vernetzten Stärke mit einem addierten gebundenen Phosphatgehalt von 0,10 bis 0,35 % für die Herstellung eines essbaren Produkts zur Kontrolle der Blutglucosekonzentration bei einem Säuger, wobei die Stärke weniger als 25 % der Glucosefreisetzung in 20 Minuten, zwischen 30-70 % in 120 Minuten und mehr als 60 % in 240 Minuten bereitstellt, und wobei die Stärke keine Stärke mit hohem Amylose-Gehalt ist, wobei die Stärke einen Gehalt von mehr als 40 % Amylose hat, die Stärke in körniger Form ist und wobei die Glucosefreisetzung nach dem Verfahren eines simulierten Verdaus, wie es in der Beschreibung offenbart ist, gemessen wird, und wobei die Stärke dextriniert oder oxidiert wurde.

2. Verwendung nach Anspruch 1, wobei die Stärke in 20 Minuten weniger als 20 % der Glucosefreisetzung bereitstellt.

3. Verwendung nach Anspruch 1, wobei die Stärke in 20 Minuten weniger als 10 % der Glucosefreisetzung bereitstellt.

4. Verwendung nach Anspruch 1, wobei die Stärke in 120 Minuten zwischen 40-60 % der Glucosefreisetzung bereitstellt.

5. Verwendung nach Anspruch 1, wobei die Stärke in 120 Minuten zwischen 45-55 % der Glucosefreisetzung bereitstellt.

6. Verwendung nach Anspruch 1, wobei die Stärke in 240 Minuten mehr als 70 % der Glucosefreisetzung bereitstellt.

7. Verwendung nach Anspruch 1, wobei die Stärke in 240 Minuten mehr als 80 % der Glucosefreisetzung bereitstellt.

8. Verwendung nach Anspruch 1, wobei die Stärke in 240 Minuten mehr als 90 % der Glucosefreisetzung bereitstellt.

9. Verwendung nach Anspruch 1, wobei die Stärke unter Verwendung eines Reagens, ausgewählt aus Natriumtrimetaphosphat, Natriumtripolyphosphat und einer Kombination davon, vernetzt ist.

10. Verwendung nach Anspruch 1, wobei die Stärke in dem essbaren Produkt in einer Menge von 5-40 % auf Trockengewichtbasis vorliegt.

11. Verwendung nach Anspruch 1, wobei die Glucosefreisetzung aus dem Nahrungsmittel im Wesentlichen nullter Ordnung ist.

12. Verwendung nach Anspruch 1, wobei die Glucosefreisetzungsrate über die ersten 240 Minuten im Wesentlichen konstant ist.

13. Verwendung nach Anspruch 1, wobei eine regulierte Glucosezufuhr für einen Säuger bereitgestellt wird.

## Revendications

1. Utilisation d'un amidon réticulé avec une teneur ajoutée en phosphate lié de 0,10 à 0,35 % pour la préparation d'un produit comestible pour maîtriser le taux de glucose dans le sang d'un mammifère, dans laquelle ledit amidon fournit moins de 25 % de la libération de glucose au bout de 20 minutes, entre 30 et 70 % au bout de 120 minutes et plus de 60 % au bout de 240 minutes, et dans laquelle ledit amidon n'est pas un amidon à teneur élevée en amylose, c'est-à-dire un amidon avec plus de 40 % d'amylose, l'amidon est sous une forme granulaire, et moyennant quoi la libération de glucose est mesurée conformément au procédé simulé de digestion tel que décrit dans la description, et l'amidon est dextrinisé ou oxydé.

2. Utilisation selon la revendication 1, dans laquelle l'amidon fournit moins de 20 % de la libération de glucose au bout de 20 minutes.

3. Utilisation selon la revendication 1, dans laquelle l'amidon fournit moins de 10 % de la libération de glucose au bout de 20 minutes.

4. Utilisation selon la revendication 1, dans laquelle l'amidon fournit entre 40 et 60 % de la libération de glucose au bout de 120 minutes.

5. Utilisation selon la revendication 1, dans laquelle l'amidon fournit entre 45 et 55 % de la libération de glucose au bout de 120 minutes.

6. Utilisation selon la revendication 1, dans laquelle l'amidon fournit plus de 70% de la libération de glucose au bout de 240 minutes.

7. Utilisation selon la revendication 1, dans laquelle l'amidon fournit plus de 80 % de la libération de glucose au bout de 240 minutes.

8. Utilisation selon la revendication 1, dans laquelle l'amidon fournit plus de 90 % de la libération de glucose au bout de 240 minutes.

9. Utilisation selon la revendication 1, dans laquelle on réticule l'amidon en utilisant un réactif choisi parmi le trimétaphosphate de sodium, le tripolyphosphate de sodium et une combinaison de ceux-ci.

10. Utilisation selon la revendication 1, dans laquelle l'amidon est présent dans le produit comestible en une quantité de 5 à 40 % sur la base du poids à sec.

11. Utilisation selon la revendication 1, dans laquelle la libération de glucose à partir de l'aliment est sensiblement d'ordre zéro.

12. Utilisation selon la revendication 1, dans laquelle le taux de libération de glucose est sensiblement constant pendant les 240 premières minutes.

13. Utilisation selon la revendication 1, dans laquelle on fournit un apport régulé en glucose à un mammifère.
